# EUROPEAN PATENT APPLICATION

(11) **EP 2 891 456 A1**
(43) Date of publication of application: **08.07.2015**
(21) Application number: 13832370.4
(22) Date of filing: 30.08.2013
(51) Int. Cl.: A61B 10/00, A61B 5/11

(54) **BRAIN FUNCTION EVALUATION SYSTEM AND BRAIN FUNCTION EVALUATION METHOD**

(30) Priority: 31.08.2012 JP 2012192339; 04.09.2012 JP 2012193894
(71) Applicant: National University Corporation Tokyo Medical and Dental University, Bunkyo-ku Tokyo 113-8510 (JP); Riken, Wako-shi, Saitama 351-0198 (JP)
(72) Inventor: ISHIKAWA, Kinya, Tokyo 113-8510 (JP); MIZUSAWA, Hidehiro, Tokyo 113-8510 (JP); NAGAO, Soichi, Saitama 351-0198 (JP); HONDA, Takeru, Saitama 351-0198 (JP); HASHIMOTO, Yuji, Tokyo 113-8510 (JP)
(74) Representative: Cabinet Plasseraud
(86) International application number: PCT/JP2013/073344
(87) International publication number: WO 2014/034856

(57) **Abstract**

In order to evaluate a motor-function disorder accompanying a brain disease by a non-conventional new method, a brain function evaluation system 1 is provided with: a display device 11 for displaying a mark indicated by a subject X; an indicated position identification unit 14 for identifying an indicated position on the display device 11 indicated by the subject X; and a divergence quantity calculation unit 16 for calculating a divergence quantity between a display position of the mark and the indicated position indicated by the subject X.

## Description

### TECHNICAL FIELD

The present invention relates to a brain function evaluation system and a brain function evaluation method, which objectively evaluate brain functions.

### BACKGROUND ART

Conventionally, in relation to brain diseases with functional movement disorders such as neurodegenerative diseases, the progression of brain diseases has generally been qualitatively comprehended by observing movement of a subject in response to oral instructions. An attempt has also been made in which a subject wears prism glasses (glasses incorporating a prism into lenses so as to refract light to deviate the sight line); the subject throws darts multiple times; and variation in the targeting accuracy (adaptation to the prism) is quantitatively tested. However, such a test is greatly influenced by the experience and ability of the medical doctor etc., and by difference in the original motor function of the subject, thereby lacking in quantifiability. Therefore, in recent years, attempts have been made to enable more quantitative evaluations of brain diseases.

For example, Patent Document 1 discloses a motor function evaluation method in which a subject indicates a mark on a display screen; the subject moves the indicated portion to a target portion; and the time required for this movement is compared with a test result of a normal healthy person, thereby evaluating the motor function.

Patent Document 1: Japanese Unexamined Patent Application, Publication No. 2004-57357

### DISCLOSURE OF THE INVENTION

### Problems to be Solved by the Invention

However, the motor function evaluation method as disclosed in Patent Document 1 requires a subject to perform many actions; therefore, some scheme for quantitatively evaluating a motor function in a simpler and more accurate manner has been desired.

Functional movement disorders may be developed as a result of cerebral diseases or cerebellar diseases. In this regard, motor function evaluation methods, which have been attempted in recent years, such as the method disclosed in Patent Document 1, involve intervention of a subject's intention (which is the intention to move the indicated portion to the target portion in Patent Document 1), and have not been able to identify whether the functional movement disorder is that of the cerebrum or cerebellum.

In particular, the present state is that qualitative evaluation methods are used as cerebellar evaluation methods, based on a physical balance, skilled motor activities of extremities, dysarthria in speech, etc.; the prism adaptation in throwing darts lacks quantifiability and accuracy, and also cannot be measured in real time; therefore, it is desired to construct a quantitative and highly accurate cerebellar evaluation method (in particular, in motor learning).

The present invention has been made in view of such a demand. A first object of the present invention is to provide a brain function evaluation system and a brain function evaluation method, which evaluate functional movement disorders related to brain diseases, through a non-conventional and novel approach. Furthermore, a second object of the present invention is to provide a brain function evaluation system and a brain function evaluation method, which are capable of accurately distinguishing cerebellar functional movement disorders, by implementing a quantitative, real-time, and highly accurate cerebellar evaluation (in particular, in motor learning functions).

### Means for Solving the Problems

A first aspect of the present invention is a brain function evaluation system, including a display device for displaying a mark to be indicated by a subject; an indicated position identification unit for identifying an indicated position on the display device indicated by the subject; and a divergence quantity calculation unit for calculating a divergence quantity between a display position of the mark and the indicated position indicated by the subject.

According to the brain function evaluation system of the first aspect, it is possible to cause the subject to indicate the mark on the display device; and it is possible to evaluate the brain function of the subject, based on the divergence quantity between the indicated position and the mark. For example, if the subject has any functional movement disorder such as a hand tremor, the divergence quantity is greater than that of a normal healthy person; therefore, the brain function of the subject can be objectively and quantitatively evaluated. As a result, the non-conventional and novel approach makes it possible to evaluate functional movement disorders that accompany brain disease.

A second aspect of the present invention is the brain function evaluation system as recited in the first aspect, further including a sight line modification unit for making a modification so that the subject's sight line deviates, in which the indicated position identification unit identifies a position indicated by the subject in a state where the sight line is modified by the sight line modification unit, and the divergence quantity calculation unit stores change in the divergence quantity that is calculated multiple times.

According to the brain function evaluation system of the second aspect, the subject indicates the mark on the display device in a state where the sight line is modified, and therefore indicates a position deviated from the mark, immediately after starting the test. A normal healthy person gradually becomes able to accurately indicate the mark through cerebellar motor learning; however, a subject with cerebellar disorders has impaired motor learning abilities, and therefore cannot accurately indicate the mark, even if the test is repeated. Further, the frequency of repetition is increased until an accurate indication is attained. Therefore, it is possible to perform a quantitative, real-time, and highly accurate evaluation of cerebellar disorders (in particular, in motor learning); and it is possible to distinguish cerebellar and cerebral functional movement disorders of the subject.

A third aspect of the present invention is the brain function evaluation system as recited in the first or second aspect, further including: a reference position detection unit for detecting that an indicator for indicating the mark is in a reference position; and a visual recognition control unit for controlling visibility of the mark; in which the visual recognition control unit makes the mark visually recognizable by the subject, on condition that the reference position detection unit detects that the indicator is in the reference position, or on condition that the indicator indicates any position on the display device; and the visual recognition control unit makes the mark visually unrecognizable by the subject after the indicator leaves the reference position and until a position is indicated on the display device.

According to the brain function evaluation system of the third aspect, when the subject separates the indicator from the reference position in an attempt to indicate the mark, the mark on the display device is made unrecognizable. Therefore, when the subject brings the indicator close to the mark (i.e. during the indicating action), the subject cannot identify the positional relationship between the indicator in motion and the mark, and cannot correct the positional deviation during the indicating action. This makes it possible to prevent the subject from correcting the indicated position by his/her intention, and to quantitatively and highly accurately evaluate the motor function by blocking the cerebral function. As a result, it is possible to accurately evaluate whether the subject has any cerebellar disorder (in particular, in motor learning); and it is possible to distinguish cerebellar and cerebral functional movement disorders in the subject.

A fourth aspect of the present invention is a brain function evaluation method, including the steps of: displaying on a display device a mark to be indicated by a subject; causing the subject to indicate the mark displayed on the display device; identifying an indicated position indicated by the subject on the display device; and calculating a divergence quantity between a display position of the mark and the indicated position indicated by the subject.

A fifth aspect of the present invention is the brain function evaluation method as recited in the fourth aspect, in which the step of causing the subject to indicate the mark is executed in a state of wearing a sight line modification unit for making a modification so that the subject's sight line deviates.

A sixth aspect of the present invention is the brain function evaluation method as recited in the fourth or fifth aspect, further including the steps of: detecting that an indicator for indicating the mark is in a reference position; making the mark visually recognizable by the subject, on condition that the indicator is detected in the reference position; making the mark visually unrecognizable by the subject, on condition that the indicator leaves the reference position; and making the mark visually recognizable by the subject, on condition that the indicator indicates any position on the display device.

According to the brain function evaluation method as recited in the fourth to sixth aspects, effects similar to the effects of the brain function evaluation system as recited in the first to third aspects are achieved.

### Effects of the Invention

According to the present invention, the non-conventional and novel approach makes it possible to determine functional movement disorders related to brain diseases, and to accurately distinguish cerebellar functional movement disorders.

### BRIEF DESCRIPTION OF THE DRAWINGS

FIG. 1 is a block diagram showing a functional configuration of a brain function evaluation system of the present invention;
FIG. 2 is a diagram showing a hardware configuration for implementing the functional configuration shown in FIG. 1;
FIG. 3 is a diagram showing procedures in a test using the brain function evaluation system;
FIG. 4 is a diagram showing an example of the test using the brain function evaluation system;
FIG. 5 is a diagram showing an example of the test using the brain function evaluation system;
FIG. 6 is a diagram showing an example of the test using the brain function evaluation system; and
FIG. 7 is a diagram showing an example of the test using the brain function evaluation system.

### PREFERRED MODE FOR CARRYING OUT THE INVENTION

An embodiment of the present invention is hereinafter described with reference to the drawings.

### Functional Configuration of Brain Function Evaluation System 1

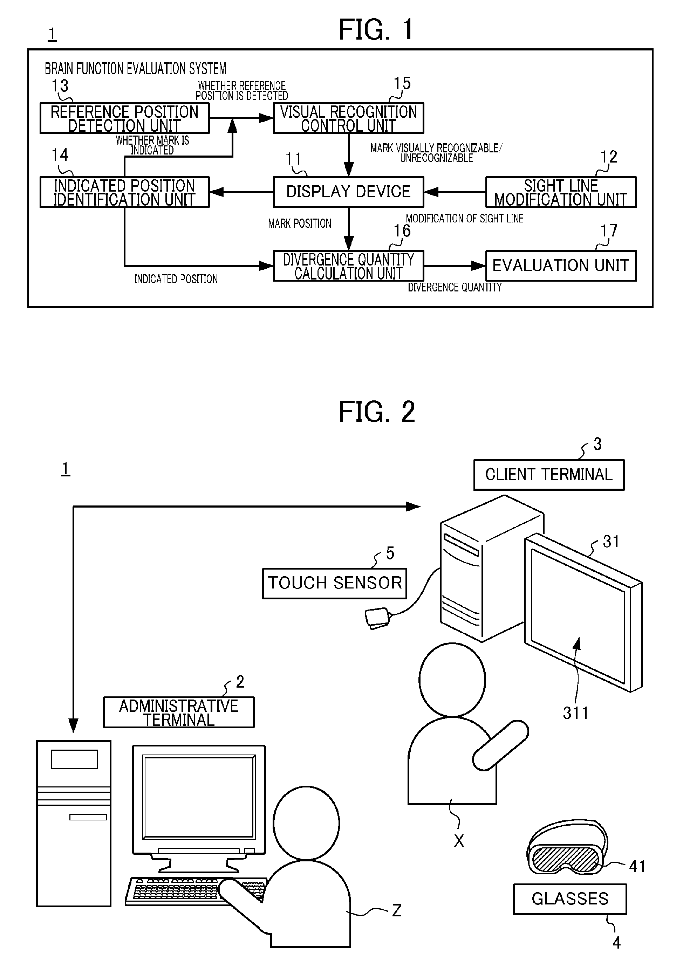

First, with reference to FIG. 1, a functional configuration of a brain function evaluation system 1 as an embodiment of the present invention is described.

The brain function evaluation system 1 is configured by including a display device 11, a sight line modification unit 12, a reference position detection unit 13, an indicated position identification unit 14, a visual recognition control unit 15, a divergence quantity calculation unit 16, and an evaluation unit 17.

The display device 11 displays a mark as a target to be indicated by a subject. The sight line modification unit 12 modifies the sight line of the subject so as to be deviated. For example, the sight line modification unit 12 modifies the sight line of the subject to the right or left at an angle within a range of 7 to 60 degrees inclusive, preferably within a range of 15 to 40 degrees inclusive.

The brain function evaluation system 1 performs a test to evaluate the brain function, as follows. In a state where the sight line is not modified (the sight line is not deviated), the subject repeats the process of indicating a mark displayed on the display device 11 with an indicator (for example, his/her finger) a predetermined number of times; and in a state where the sight line is modified (the sight line is deviated), the subject repeats the process of indicating a mark displayed on the display device 11 with the indicator (for example, his/her finger) a predetermined number of times.

The reference position detection unit 13 detects whether the indicator is in the reference position, and notifies the visual recognition control unit 15 of the result.

The indicated position identification unit 14 detects that an arbitrary position on the display device is indicated by the subject, and notifies the visual recognition control unit 15 of the result. The indicated position identification unit 14 identifies a position indicated by the subject on the display device (hereinafter referred to as "indicated position"), and notifies the divergence quantity calculation unit 16 of the indicated position thus identified. For example, coordinates on the display device 11 can be employed as the indicated position.

The visual recognition control unit 15 controls the mark displayed on the display device 11 to be visually recognizable or unrecognizable by the subject. The display device 11 displays the mark in an arbitrary position (hereinafter referred to as "display position"). For example, the display device 11 determines a display position at random a predetermined number of times, and displays the mark in the display position thus determined.

When the indicator is in the reference position, the visual recognition control unit 15 controls the mark displayed on the display device 11 to be visually recognizable by the subject. When the indicator leaves the reference position, the visual recognition control unit 15 controls the mark displayed on the display device 11 to be visually unrecognizable by the subject. When the indicator subsequently indicates a position on the display device 11, the visual recognition control unit 15 controls the mark, which was visually unrecognizable, to be visually recognizable again by the subject.

The display device 11 notifies the divergence quantity calculation unit 16 of the display position of the mark. For example, coordinates on the display device 11 can be employed as the display position.

The divergence quantity calculation unit 16 calculates a divergence quantity between the display position of the mark and the subject's indicated position, i.e. a divergence quantity between the coordinates showing the display position and the coordinates showing the indicated position (the distance between the two coordinates), and notifies the evaluation unit 17 of the calculation result.

The evaluation unit 17 evaluates the degree of divergence, based on the divergence quantity calculated by the divergence quantity calculation unit 16 (for example, through comparison with statistics of the normal healthy person's divergence quantity). As will be described later in detail concerning evaluation, when the subject's divergence quantity is large (e.g. when the absolute value is large, or when the relative value is large in comparison with statistics of the normal healthy person's divergence quantity), the evaluation unit 17 displays the divergence quantity (by print or screen output, etc.) to make it possible to evaluate that the divergence quantity significantly differs from the normal healthy person's divergence quantity. At this time, the evaluation unit 17 also displays tendency in terms of change in the divergence quantity when the test is repeated.

### Specific Configuration of Brain Evaluation System 1

Next, with reference to FIG. 2, descriptions are provided for a hardware configuration for specifically implementing the functional configuration shown in FIG. 1. Note that the configuration shown in FIG. 2 is merely an example, and may be implemented through other configurations, as long as the function shown in FIG. 1 can be achieved.

As shown in FIG. 2, the brain function evaluation system 1 is configured by including an administrative terminal 2, a client terminal 3, glasses 4, and a touch sensor 5.

The administrative terminal 2 is a terminal device, which is used by an administrator Z (for example, a doctor, a test engineer, or the like) who administers the test with the brain function evaluation system 1, and the administrative terminal 2 is communicatively connected to the client terminal 3. The administrative terminal 2 is installed with an administrative program for performing a test, and operates in accordance with the manager administrative program executed by the administrator Z, thereby implementing various functions such as determining display position, calculating a divergence quantity, and comparing with a normal healthy person's statistics.

The client terminal 3 is a terminal device which is set up to face a subject X to be tested and is configured to include a display 31. The display 31 is a liquid crystal display which is controlled by the client terminal 3 to display a mark for the subject X. A touchscreen 311 is arranged on the front face of the display 31, which is configured to be capable of identifying an indicated position indicated by the subject X.

A prism lens 41 can be arranged on the front face side of the glasses 4, which the subject X wears. The prism lens 41 is a removable plate-like prism lens, which can be inserted into and removed from the left side face of the glasses 4, and makes a modification so that the sight line of the subject X deviates. An electromagnetic shutter, which becomes transparent or opaque depending on whether voltage is applied thereto, is incorporated in the foreground of the glasses 4. The electromagnetic shutter allows the mark displayed on the display 31 to be visually recognizable or unrecognizable by the subject X.

The touch sensor 5 detects whether the sensor is touched by the subject X, and notifies the detection result to the client terminal 3 and the glasses 4, which are connected through a USB (Universal Serial Bus) or a wireless LAN. In the present embodiment, the ear-cuff touch sensor 5 is used. More specifically, the reference position in the present embodiment refers to a position of the touch sensor 5, which is attached to the ear.

In the brain function evaluation system 1 with such a configuration, the client terminal 3 (display 31) functions as the display device 11, and the glasses 4, with the prism lens 41 inserted, function as the sight line modification unit 12. More specifically, the sight line of the subject X who wears the glasses 4 is modified by the prism lens 41 inserted in the front face side of the glasses 4. As a result, the subject X sees the mark displayed on the display 31 in a position deviated from the actual position. Here, the prism lens 41 modifies the sight line of the subject to the right or left at an angle within a range of 7 to 60 degrees inclusive, preferably within a range of 15 to 40 degrees inclusive. If the angle of modification is less than 15 degrees (in particular, less than 7 degrees), the deviation of the sight line is too small to serve as a substantial modification; and if the angle of modification is larger than 40 degrees (in particular, larger than 60 degrees), the learning limit of the motor function is exceeded, both angles making it difficult to evaluate the cerebellar function.

In the brain function evaluation system 1, the touch sensor 5 functions as the reference position detection unit 13; and the touchscreen 311 functions as the indicated position identification unit 14. More specifically, when the touch sensor 5 attached to the ear is touched by a finger of the subject X, the touch sensor 5 detects that the indicator (the finger of the subject X) is in the reference position; and when the finger is released from the touch sensor 5, the touch sensor 5 detects that the indicator (the finger of the subject X) is not in the reference position. The position where the finger of the subject X touches the touchscreen 311, which is arranged on the front face of the display 31 displaying the mark, is identified as an indicated position indicated by the subject X.

In the brain function evaluation system 1, the electromagnetic shutter of the glasses 4 functions as the visual recognition control unit 15. More specifically, when the administrative terminal 2 determines a display position for displaying a mark at random, and notifies the client terminal 3 of the display position, the client terminal 3 displays the mark in the display position of the display 31, in accordance with this notification.

The electromagnetic shutter of the glasses 4 is communicatively connected to the touch sensor 5 and the touchscreen 311. In a state where the touch sensor 5 detects a finger touch, the electromagnetic shutter allows the mark to be visually recognized by the subject X; and in a state where the touch sensor 5 does not detect a finger touch, the electromagnetic shutter does not allow the mark to be visually recognized by the subject X until the touchscreen 311 detects a finger touch thereafter.

In the brain function evaluation system 1, the administrative terminal 2 functions as the divergence quantity calculation unit 16 and the evaluation unit 17. More specifically, the administrative terminal 2 receives the indicated position identified by the touchscreen 311 from the client terminal 3, and compares the indicated position with the display position of the mark, thereby calculating how much the indicated position indicated by the subject X diverges from the display position of the mark. The client terminal 3 outputs tendency in terms of the divergence quantity obtained in the tests performed a predetermined number of times (by screen display or printout) to make it possible to evaluate that the divergence quantity significantly differs from a normal healthy person's divergence quantity, such that the administrator Z can make a judgment on the brain function of the subject X.

### Test Method of Brain Evaluation System 1

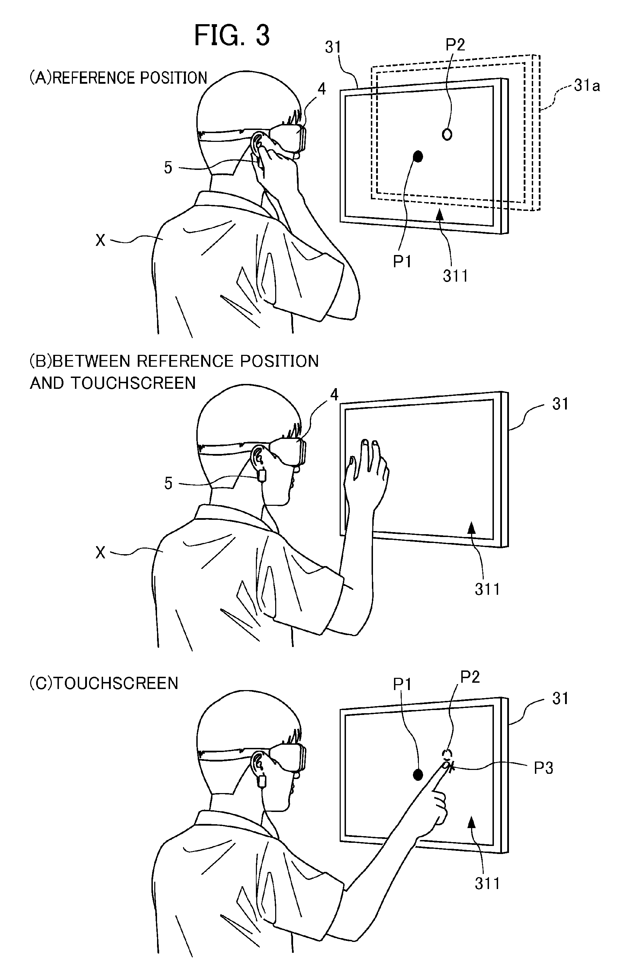

The configuration of the brain function evaluation system 1 of the present embodiment has been described above. Next, with reference to FIG. 3, descriptions are provided for procedures of the test using the brain function evaluation system 1.

The subject X waits in a position where his/her finger can touch the touchscreen 311 with a moderate pressure (for example, a position at a distance of about 50 cm from the display 31, the position appropriately adjusted to the subject X's arm length), and wears the glasses 4 and the touch sensor 5. The subject X sits and waits in a state where he/she is putting his/her chin on a rest (illustration omitted). Here, in the present embodiment, the test in a state where the sight line is not modified, and the test in a state where the sight line is modified, are each repeated a predetermined number of times. For this purpose, a dummy transparent acrylic board which does not modify the sight line, or the prism lens 41 which modifies the sight line, is inserted into the glasses 4, as necessary. In FIG. 3, the subject X wears the glasses 4 with the prism lens 41 inserted; and the sight line of the subject X is modified so as to be deviated 25 degrees to the right.

As shown in FIG. 3A, when the subject X touches the touch sensor 5 with his/her finger, a mark is displayed on the display 31, in a display position P1 indicated by the administrative terminal 2. At this time, in FIG. 3, since the sight line of the subject X is modified, the subject X sees the mark as if it is displayed in a modification position P2, which is deviated 25 degrees to the right from the actual display position P1. In FIG. 3A, a display 31a and the modification position P2 are displayed on the upper right of the display 31 and the display position P1, respectively, with the description taking into consideration that the subject X to be tested in a dark place may incline his/her head.

Subsequently, the subject X releases his/her finger from the touch sensor 5, and indicates a mark on the touchscreen 311 with his/her finger, thereby performing the test. At this time, in order to prevent the subject X from consciously adjusting the finger position toward the mark during the indicating action, the subject X is supposed to perform the indicating action at a constant speed in a rhythmic fashion.

As shown in FIG. 3B, when the subject X releases his/her finger from the touch sensor 5, the function of the electromagnetic shutter of the glasses 4 disables the subject X from visually recognizing the mark on the display 31. This makes it difficult for the subject X to consciously adjust the finger position toward the mark during the indicating action, and makes it possible to prevent the subject X from making an intentional adjustment. In other words, this blocks the cerebral function.

Subsequently, when the subject X indicates a position on the touchscreen 311 with his/her finger (the subject X's finger touches the touchscreen 311), the mark on the display 31 becomes visually recognizable again by the subject X, as shown in FIG. 3C. At this time, in FIG. 3, since the sight line of the subject X is modified, the subject X will indicate an indicated position P3 in the vicinity of the modification position P2, instead of the display position P1. The indicated position P3 and the display position P1 are transmitted to the administrative terminal 2, which calculates a divergence quantity (distance) between the indicated position P3 and the display position P1.

Subsequently, when the subject X releases his/her finger from the touchscreen 311, and touches the touch sensor 5 with his/her finger, a mark for the next test is displayed on the display 31, in a display position different from the previous position, in which the mark is visually recognizable by the subject X. In the test using the brain function evaluation system 1, such an action of indicating the mark is repeated to make a judgment on the brain function of the subject X, based on the divergence quantity between the indicated position P3 indicated by the subject X and the display position P1, more particularly, based on the tendency in terms of change in the divergence quantity.

### Example

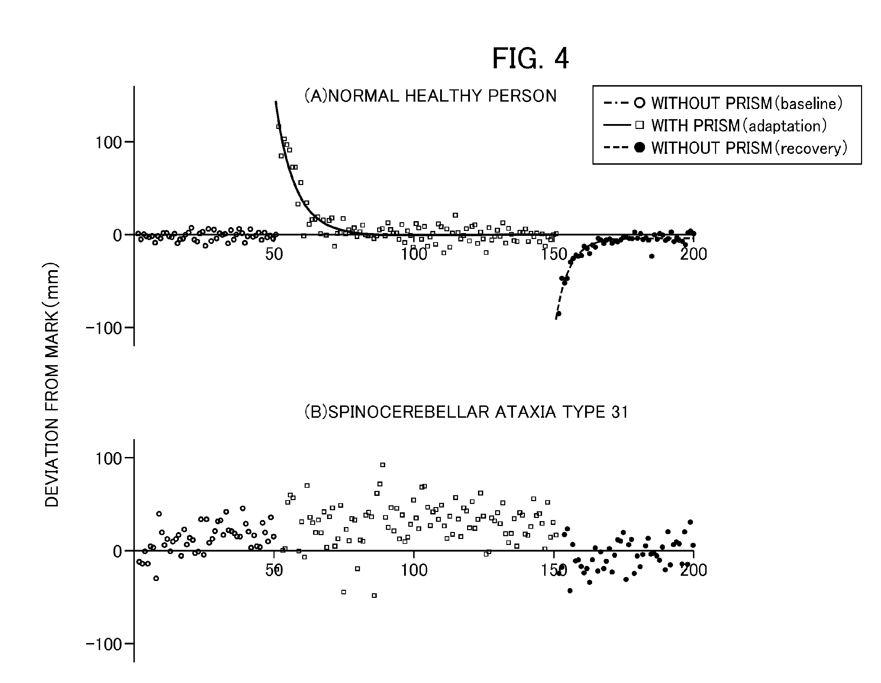

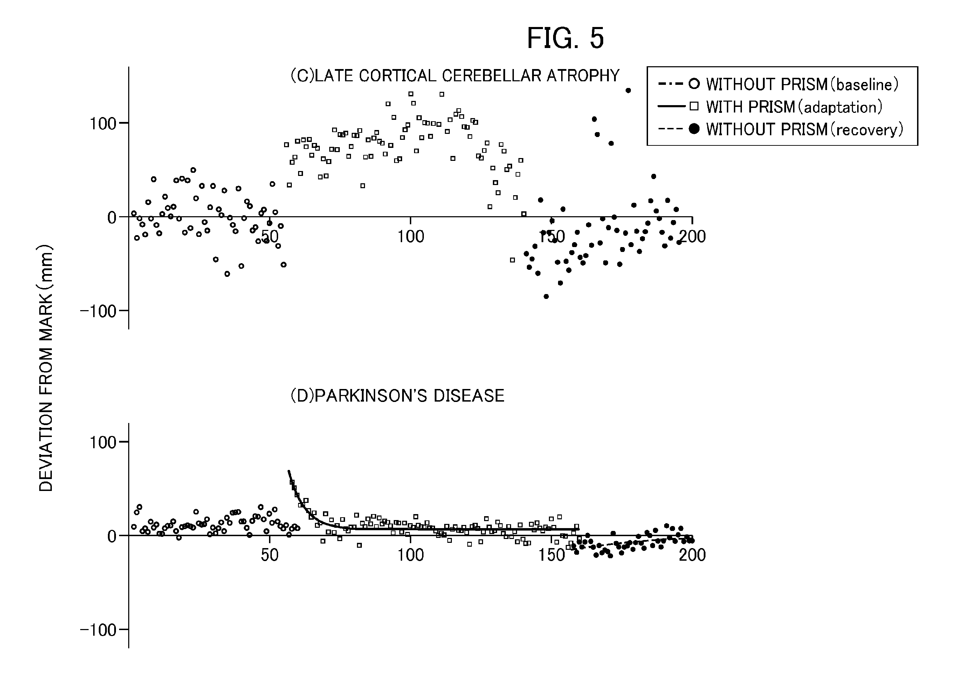

Next, an example of the test using the brain function evaluation system 1 is described with reference to FIGS. 4 and 5. The inventors of the present invention performed tests using the brain function evaluation system 1, in which subjects included: a normal healthy person (FIG. 4A), a patient with spinocerebellar ataxia type 31 (FIG. 4B), a patient with late cortical cerebellar atrophy (FIG. 5C), and a patient with Parkinson's disease (FIG. 5D). Among the subjects, the normal healthy person is a subject without cerebral/cerebellar disorders; the patient with spinocerebellar ataxia type 31 and the patient with late cortical cerebellar atrophy are subjects with cerebellar disorders; and the patient with Parkinson's disease is a subject without cerebellar disorders but with cerebral disorders.

The test in the example was performed by: repeating the test 50 times in a state where the subjects wore the glasses 4 with the dummy transparent acrylic board inserted in place of the prism lens 41; repeating the test 100 times in a state where the subjects wore the glasses 4 with the prism lens 41 inserted to deviate the sight line 25 degrees to the right; and repeating the test 50 times in a state where the subjects wore the glasses 4 with the dummy transparent acrylic board inserted again. The dummy transparent acrylic board was used for the purpose of preventing the subjects from knowing whether the sight line was modified.

First of all, an example of the normal healthy person is described with reference to FIG. 4A.

In the first 50 tests, the prism lens 41 was not inserted, and the dummy transparent acrylic board did not modify the sight line; therefore, the normal healthy person showed a tendency to indicate positions in the vicinity of the mark.

In the subsequent 100 tests, the normal healthy person firstly indicated positions deviated from the mark, but showed a tendency to gradually indicate positions in the vicinity of the mark, as the test was repeated. The sight line was modified by the prism lens 41, and the subject firstly indicated positions deviated by a quantity equal to the modification, but then became able to accurately indicate the mark, which is considered to be attributable to the cerebellar motor learning function having worked, as the test was subsequently repeated.

In the subsequent 50 tests, the inserted prism lens 41 was removed and replaced with the dummy transparent acrylic board to cancel the modification of the sight line. Therefore, the normal healthy person firstly indicated positions deviated from the mark by a quantity equal to the quantity learned by the cerebellum, but as the test was repeated, the learning function worked again, and the normal healthy person showed a tendency to indicate positions which gradually got closer to the mark.

Next, an example of the patient with spinocerebellar ataxia type 31 is described with reference to FIG. 4B.

In the first 50 tests, the subject showed a tendency to indicate discrete positions deviated from the mark, although the sight line was not modified. This is considered to be attributable to the functional movement disorder of the subject.

In the subsequent 100 tests, the sight line was modified, and although the subject was not free from a tendency to indicate positions deviated from the mark by a quantity influenced by the modification, the subject still indicated discrete positions, which did not get closer to the vicinity of the mark, even if the test was repeated. This is considered to be attributable to the cerebellar disorder, which disables the motor learning function.

In the subsequent 50 tests, the modification of the sight line was cancelled, but the subject still showed a tendency to indicate discrete positions deviated from the mark, similarly to the first 50 tests. The subject did not show any tendency to deviate in a direction opposite to the initial modification quantity, which was observed in the normal healthy person. This is considered to be attributable to the motor learning function having not worked in the 100 tests, as a result of which the subject did not indicate positions deviated by a learned amount, which was observed in the normal healthy person.

As shown in FIG. 5C, the patient with late cortical cerebellar atrophy with cerebellar disorders showed a tendency similar to the patient with spinocerebellar ataxia type 31. In other words, the subject showed a tendency to indicate discrete positions deviated from the mark, even if the sight line was not modified. In a state where the sight line was modified, the subject indicated positions deviated from the mark by a quantity equal to the modification, but the indicated positions did not get closer to the vicinity of the mark even if the test was repeated.

Next, an example of the patient with Parkinson's disease with cerebral disorders is described with reference to FIG. 5D.

In the first 50 tests, the subject showed a tendency to indicate positions that deviated from the mark, even if the sight line was not modified, but the degree of variation was smaller than the patient with spinocerebellar ataxia type 31 and the patient with late cortical cerebellar atrophy. This is considered to be attributable to a non-cerebellar functional movement disorder of the subject.

On the other hand, in the subsequent 100 tests the subject firstly indicated positions that deviated by a quantity equal to the modification, but showed a tendency to become basically able to indicate the mark, as the test was subsequently repeated, similar to the case of the normal healthy person. This is considered be attributable to the cerebellar motor learning function having worked through repetition, as a result of which the subject became able to indicate the mark.

In the subsequent 50 tests as well, as in the case of the normal healthy person, the subject firstly indicated positions that deviated from the mark by a quantity equal to the quantity learned by the cerebellum, but as the test was repeated, the learning function worked again, and the subject showed a tendency to indicate positions that gradually got closer to the mark.

The examples as described above have revealed that it is possible to determine whether a subject has any functional movement disorder related to cerebral or cerebellar disorders, by causing the subject to indicate the mark displayed on the display 31, and by observing the tendency in terms of change in the divergence quantity. More specifically, as shown in the first 50 tests, the subjects with functional movement disorders show a tendency not to be able to accurately indicate the mark, unlike the normal healthy person. Therefore, by observing the divergence quantity, it is possible to quantitatively evaluate and determine whether the subject has any functional movement disorder.

The examples have also revealed that it is possible to determine whether a subject has any functional movement disorder related to cerebellar disorders, by causing the subject to indicate the mark with the sight line modified, and by observing the tendency in terms of change in the divergence quantity. In other words, as shown in the subsequent 100 tests, even if the sight line was modified, the subjects without cerebellar disorders, whose motor learning function works, gradually become able to accurately indicate the mark, as the test is repeated; whereas the subject with cerebellar disorders, whose motor learning function does not work, do not show any improvement in the divergence quantity, even if the test is repeated. Also, as shown in the subsequent 50 tests, when the modification of the sight line is cancelled, the subjects without cerebellar disorders show a tendency to firstly indicate positions that deviated from the mark by a learned amount, and then to gradually indicate positions accurately; whereas the subjects with cerebellar disorders do not show any tendency to indicate positions deviating in a direction opposite to the modification quantity accompanying learning the modified sight line, and do not show any change in the divergence quantity, even if the test is subsequently repeated. Therefore, it is possible distinguish cerebellar functional movement disorders of the subject, by modifying the sight line and causing the subject to indicate the mark.

### Additional Example

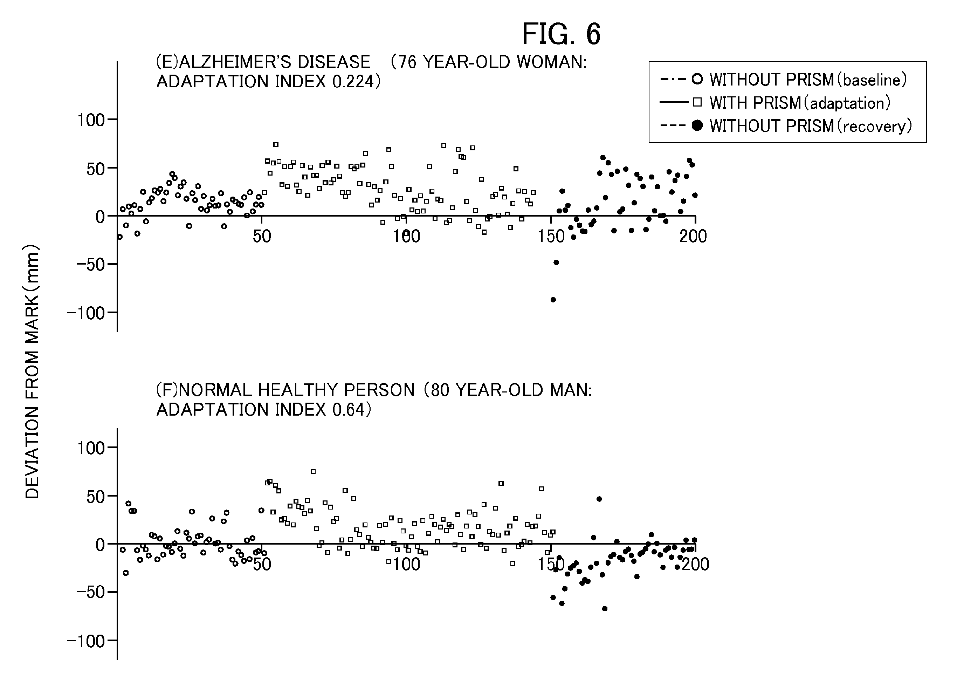

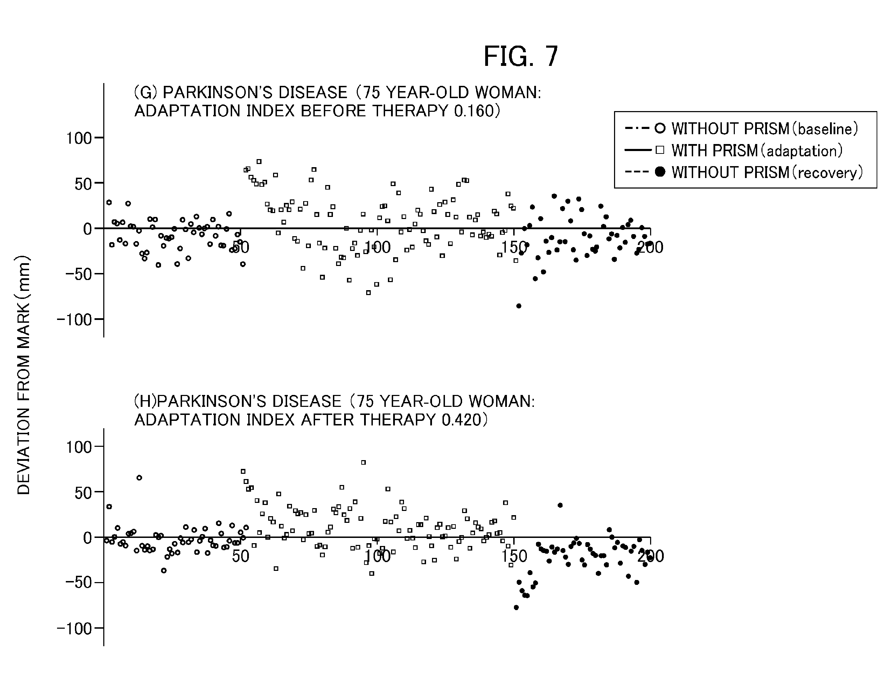

Next, an example of an additional test using the brain function evaluation system 1 is described with reference to FIGS. 6 and 7. A method for the additional test is identical to the method for the test shown in FIGS. 4 and 5. FIGS. 6 and 7 show an adaptation index for each subject. The adaptation index shows comparison with a test result for an arbitrary normal healthy person (for example, the normal healthy person of FIG. 4A), and shows that a subject is more normal and healthy as the adaptation index becomes closer to "1".

The inventors of the present invention performed tests using the brain function evaluation system 1, in which the subjects included a patient with Alzheimer's disease (FIG. 6E), an aged normal healthy person (FIG. 6F), and a patient with Parkinson's disease (FIG. 7). Among these subjects, the patient with Alzheimer's disease is a subject with memory deterioration but without general cerebellar disorders; and the aged normal healthy person is a subject (the spouse) who is older than this patient with Alzheimer's disease.

FIG. 6 shows a comparison between the patient with Alzheimer's disease (FIG. 6E) and the normal healthy person (FIG. 6F) who is older than this patient. FIG. 7 shows a comparison between a pre-therapeutic level (FIG. 7G) and a post-therapeutic level (FIG. 7H) of the patient with Parkinson's disease. Namely, FIGS. 7G and 7H show the tests for the same patient. More specifically, FIG. 7G shows a test result at an initial diagnosis phase when no medication was administered; and FIG. 7H shows a test result at a point in time when the patient's life was made easier by taking some medicine for Parkinson's disease for several months.

With reference to FIG. 6, comparing the adaptation indexes between the patient with Alzheimer's disease and the subject older than the patient, the adaptation index of the patient with Alzheimer's disease is lower. This is considered to be attributable to the low ability of the patient with Alzheimer's disease to memorize the mark displayed on the display 31.

From this, according to the brain function evaluation system 1, it is possible to determine the quality of human memory function, which is called a working memory. That is to say, since the additional test has revealed that the patient with Alzheimer's disease shows a deteriorated result, the brain function evaluation system 1 can be applied to a test for dementia such as Alzheimer's disease.

With reference to FIG. 7, in the test performed before and after therapy for the patient with Parkinson's disease, the low adaptation index before therapy was remarkably improved after therapy. That is to say, overall variability decreased, and movement improved. With reference to FIG. 7, it is understood that the symptoms of Parkinson's disease, which were improved by therapy, is reflected in the test result.

From this, the brain function evaluation system 1 can be preferably used for evaluation and pharmacometrics of symptoms of the Parkinson's disease. It is also possible to quantitatively evaluate hand tremors, etc. of a subject.

According to the brain function evaluation system 1 described above, the brain function of the subject is evaluated, based on the divergence quantity between the mark and the position indicated by the subject. For example, if a subject has any functional movement disorder such as hand tremors, the divergence quantity increases; therefore, the brain function of the subject can be objectively and quantitatively evaluated through comparison with the normal healthy person's result. As a result, this non-conventional and novel approach makes it possible to determine whether a subject has any functional movement disorder related to a brain disease.

The brain function evaluation system 1 evaluates the brain function of the subject by comparing the tendency in terms of change in the divergence quantity between the indicated position and the mark, in relation to the subject and the normal healthy person, in a state where the sight line is modified. The normal healthy person without cerebellar disorders gradually becomes able to indicate the mark through the cerebellar motor learning function, even if the sight line is modified; whereas the subject with cerebellar disorders has a deteriorated ability of motor learning, and cannot accurately indicate the mark, even if the test is repeated. Therefore, the cerebellum can be quantitatively evaluated, thereby making it possible to distinguish whether the brain disease is cerebellar or cerebral.

As an example, the system can be utilized for diagnosis of cerebellar diseases such as spinocerebellar ataxia type 31 and spinocerebellar degeneration, and can distinguish extrapyramidal diseases, such as Parkinson's disease and essential tremor, which do not impair the cerebellum, from extrapyramidal diseases such as multiple system atrophy, which is accompanied by symptoms of Parkinson's disease and could also impair the cerebellum. The usefulness of the brain function evaluation system 1 can be demonstrated in daily clinical practice, in which these two types of diseases are sometimes difficult to distinguish if a subject has an early-stage disease or complications, etc. Furthermore, the system is useful for excluding cerebellar disorders in diagnosing symptoms similar to cerebellar disorders, even in cases of, for example, atactic hemiparesis caused by cerebrovascular disorders or multiple sclerosis, which are difficult to distinguish by way of medical examinations and MRI. That is to say, the system can be widely applied to identifying a multitude of diseases, brain development and aging, as well as diseases attacking the cerebellum.

Midway through the process of indicating the mark by the subject, the brain function evaluation system 1 causes the mark on the display 31 to be visually unrecognizable. Therefore, when a finger is brought close to the mark, the subject can no longer identify the positional relationship between the finger in motion and the mark, and therefore cannot correct the positional deviation during the indicating action. This makes it possible to prevent the subject from correcting the indicated position by his/her intention, and to quantitatively evaluate the cerebellar motor function by blocking the cerebral function.

The brain function evaluation system 1 can be widely utilized for elucidating brain functions, such as evaluating functional linkage inside the brain, and pediatric brain maturation. Furthermore, the brain function evaluation system 1 can be utilized for functional evaluations of an autistic spectrum disorder, which is considered to surely involve the cerebellum.

The embodiment of the present invention has been described above; however, the present invention is not limited to the embodiment described above. The effects disclosed in the present embodiment are only a list of the most preferred effects achieved by the invention; and the effects of the invention are not limited to those disclosed in the embodiment.

For example, in the embodiment described above, the visual recognition control unit 15 implements the function of controlling the mark to be visually recognizable or unrecognizable; however, the present invention is not limited thereto. For example, a physical shutter may be provided on the front face of the display device 11 (display 31) to physically make the mark visually unrecognizable by the subject. A so-called normally white liquid crystal display, whose screen turns white when no voltage is applied, may be used to implement this function, such that the mark is displayed or not displayed on the liquid crystal display, in accordance with indication from the administrative terminal 2 and the client terminal 3.

Furthermore, in the embodiment described above, the dummy transparent acrylic board, the prism lens 41 deviating from the sight line by 25 degrees to the right, and the dummy transparent acrylic board are inserted into the glasses 4 in this order to perform the test; however, the present invention is not limited thereto. For example, prism lenses 41 for deviating from the sight line in an opposite direction may be inserted hallway, such that the dummy transparent acrylic board, the prism lens 41 deviating the sight line by 25 degrees to the right, the prism lens 41 deviating the sight line by 25 degrees to the left, and the dummy transparent acrylic board are inserted in this order. In this case, the prism lens 41 for deviating from the sight line in an opposite direction can create a more remarkable tendency than the dummy transparent acrylic board can. The deviation angle for the sight line is not limited to 25 degrees; similar test results can be obtained at a deviation angle of 15 degrees or 40 degrees, and a practical test can be performed within a range of 7 to 60 degrees inclusive.

### EXPLANATION OF REFERENCE NUMERALS

- 1: brain function evaluation system
- 11: display device
- 12: sight line modification unit
- 13: reference position detection unit
- 14: indicated position identification unit
- 15: visual recognition control unit
- 16: divergence quantity calculation unit
- 17: evaluation unit
- 2: administrative terminal
- 3: client terminal
- 31: display
- 311: touchscreen
- 4: glasses
- 41: prism lens
- 5: touch sensor

## Claims

1. A brain function evaluation system, comprising:
a display device for displaying a mark to be indicated by a subject;
an indicated position identification unit for identifying an indicated position on the display device indicated by the subject; and
a divergence quantity calculation unit for calculating a divergence quantity between a display position of the mark and the indicated position indicated by the subject.

2. The brain function evaluation system according to claim 1, further comprising:
a sight line modification unit for making a modification so that the subject's sight line deviates;
wherein the indicated position identification unit identifies the indicated position indicated by the subject in a state where the sight line is modified by the sight line modification unit; and
wherein the divergence quantity calculation unit stores change in the divergence quantity that is calculated multiple times.

3. The brain function evaluation system according to claim 1 or 2, further comprising:
a reference position detection unit for detecting that an indicator for indicating the mark is in a reference position; and
a visual recognition control unit for controlling visibility of the mark;
wherein the visual recognition control unit makes the mark visually recognizable by the subject, on condition that the reference position detection unit detects that the indicator is in the reference position, or on condition that the indicator indicates any position on the display device; and the visual recognition control unit makes the mark visually unrecognizable by the subject, after the indicator leaves the reference position and until a position is indicated on the display device.

4. A brain function evaluation method, comprising the steps of:
displaying on a display device a mark to be indicated by a subject;
causing the subject to indicate the mark displayed on the display device;
identifying an indicated position indicated by the subject on the display device; and
calculating a divergence quantity between a display position of the mark and the indicated position indicated by the subject.

5. The brain function evaluation method according to claim 4,
wherein the step of causing the subject to indicate the mark is executed in a state of wearing a sight line modification unit for making a modification so that the subject's sight line deviates.

6. The brain function evaluation method according to claim 4 or 5, further comprising the steps of:
detecting that an indicator for indicating the mark is in a reference position;
making the mark visually recognizable by the subject, on condition that the indicator is detected in the reference position;
making the mark visually unrecognizable by the subject, on condition that the indicator leaves the reference position; and
making the mark visually recognizable by the subject, on condition that the indicator indicates any position on the display device.

7. A brain function evaluation system, comprising:
a display device for displaying a mark to be indicated by a subject;
a reference position detection unit for detecting that the subject's fingertip for indicating the mark is in a reference position on his/her ear;
an indicated position identification unit for identifying an indicated position on the display device indicated by the subject;
a divergence quantity calculation unit for calculating a divergence quantity between a display position of the mark and an indicated position indicated by the subject;
a sight line modification unit for making a modification so that the subject's sight line deviates; and
a visual recognition control unit for controlling visibility of the mark;
wherein the visual recognition control unit makes the mark visually recognizable by the subject, on condition that the reference position detection unit detects that the subject's fingertip is in the reference position; and the visual recognition control unit makes the mark visually unrecognizable by the subject, on condition that the subject's fingertip leaves the reference position;
wherein the indicated position identification unit identifies the indicated position indicated by the subject in a state where the sight line is modified by the sight line modification unit; and
wherein the divergence quantity calculation unit stores change in the divergence quantity that is calculated multiple times.

8. The brain function evaluation system according to claim 7,
wherein the visual recognition control unit makes the mark visually unrecognizable by the subject, after the subject's fingertip leaves the reference position and until a position is indicated on the display device; and the visual recognition control unit makes the mark visually recognizable by the subject, on condition that the subject's fingertip indicates any position on the display device.

9. A brain function evaluation method, comprising the steps of:
displaying on a display device a mark to be indicated by a subject;
detecting that the subject's fingertip for indicating the mark is in a reference position on his/her ear;
causing the subject to indicate the mark displayed on the display device;
making the mark visually recognizable by the subject, on condition that the subject's fingertip is detected in the reference position;
making the mark visually unrecognizable by the subject, on condition that the subject's fingertip leaves the reference position;
identifying an indicated position indicated by the subject on the display device; and
calculating a divergence quantity between a display position of the mark and the indicated position indicated by the subject;
wherein the step of causing the subject to indicate the mark is executed in a state of wearing a sight line modification unit for making a modification so that the subject's sight line deviates.

10. The brain function evaluation method according to claim 9, further comprising:
making the mark visually recognizable by the subject, on condition that the subject's fingertip indicates any position on the display device.
